# EUROPÄISCHE PATENTSCHRIFT

(11) **EP 2 844 213 B1**
(45) Veröffentlichungstag und Bekanntmachung des Hinweises auf die Patenterteilung: **04.12.2019**
(21) Anmeldenummer: 13720870.8
(22) Anmeldetag: 25.04.2013
(51) Int. Cl.: C03C 3/097, C03C 10/00

(54) **LITHIUMDISILIKAT-APATIT-GLASKERAMIK**
LITHIUM DISILICATE APATITE GLASS CERAMIC
VITROCÉRAMIQUE À L'APATITE ET AU LITHIUM-SILICE

(30) Priorität: 04.05.2012 EP 12166760
(43) Veröffentlichungstag der Anmeldung: 11.03.2015
(73) Patentinhaber: Ivoclar Vivadent AG, 9494 Schaan (LI)
(72) Erfinder: RITZBERGER, Christian, CH-9472 Grabs (CH); HOELAND, Wolfram, 9494 Schaan (LI); SCHWEIGER, Marcel, CH-7000 Chur (CH); RHEINBERGER, Volker, 9490 Vaduz (LI)
(74) Vertreter: Uexküll & Stolberg
(86) Internationale Anmeldenummer: PCT/EP2013/058672
(87) Internationale Veröffentlichungsnummer: WO 2013/164256

(56) Entgegenhaltungen:
- EP-A2- 0 885 855
- EP-A2- 0 885 856
- WO-A1-2007/028787
- US-A- 5 804 520
- US-A1- 2008 120 994
- US-B1- 6 455 451
- MARTIN PALOU: "Mechanism and kinetics of glass-ceramics formation in the Li2O-SiO2-CaO-P2O5-CaF2 system", CENTRAL EUROPEAN JOURNAL OF CHEMISTRY, Bd. 7(2), 2009, Seiten 228-233, XP002682499, Versita Warsaw, springer Berlin
- W.HÖLLAND, E. APPEL, CH. VAN'T HOEN, V. RHEINBEREGER: "Studies of crystal phase formations in high-strength lithium dislicate glass-ceramics", JOURNAL OF NON-CRYSTALLINE SOLIDS, Bd. 352, 14. September 2006 (2006-09-14), Seiten 4041-4050, XP002682500,
- W.HÖLAND, V.RHEINBERGER, M.FRANK: "Mechanisms of nucleation and controlled crystallisation of needle-like apatite in glass-ceramics of the type Si02-Al203-K20-Ca0-P205 systems", JOURNAL OF NON-CRYSTALLINE SOLIDS, Bd. 253, 1. Januar 1999 (1999-01-01), Seiten 170-177, XP002682501,

## Beschreibung

Die Erfindung betrifft Lithiumdisilikat-Apatit-Glaskeramik, die sich insbesondere zum Einsatz in der Zahnheilkunde, bevorzugt zur Herstellung von dentalen Restaurationen, eignet sowie Vorstufen zu deren Herstellung.

Glaskeramiken mit einer Lithiumdisilikat- und einer Apatit-Kristallphase sind aus dem Stand der Technik bekannt.

M. Palou et al. berichten in Cent. Eur. J. Chem, 7(2), 228-233 (2009) über die Kristallisation von einer Mischung aus reinem Lithiumdisilikat-Glas und Fluorapatit-Glas. Die dabei erzeugte Glaskeramik hat einen hohen Gehalt von 14 Gew.-% P₂O₅ und zeigt Bioaktivität beim in-vitro Test in simulierter Körperflüssigkeit.

S.C. Mojumdar et al. beschreiben in Journal of Thermal Analysis and Calorimetry 78(1), 73-82 (2004) Untersuchungen zur Kristallisation von Gläsern aus dem System Li₂O-CaO-CaF₂-P₂O₅-SiO₂ mit unterschiedlichen Gehalten an P₂O₅. Nach Kristallisation eines Glases mit einem Gehalt von 15 Gew.-% P₂O₅ wurde neben einer Lithiumdisilikat-Kristallphase auch Fluorapatit mittels Röntgenbeugung nachgewiesen.

Bei den aus dem Stand der Technik bekannten Lithiumsilikat-Glaskeramiken mit Apatit-Kristallphase handelt es sich jedoch um bioaktive Produkte und nicht um chemisch beständige Materialien, die für die restaurative Zahnmedizin geeignet sind. Bioaktive Produkte bilden in Körperflüssigkeiten oder simulierten Körperflüssigkeiten Apatitkristalle auf der Oberfläche aus, um z.B. im Falle eines endoprothetischen Implantats einen festen Verbund mit dem Knochen zu ergeben.

Die bekannten Glaskeramiken leiden daher an dem schwerwiegenden Nachteil, dass sie nicht über die chemische Beständigkeit verfügen, die für ein Dentalmaterial erforderlich ist, welches in Kontakt mit verschiedensten Fluiden in der Mundhöhle kommt.

Der Erfindung liegt daher die Aufgabe zugrunde, eine Lithiumdisilikat-Apatit-Glaskeramik zur Verfügung zu stellen, die über eine sehr gute chemische Beständigkeit verfügt und damit als restauratives Dentalmaterial verwendet werden kann. Die Glaskeramik soll darüber hinaus in einfacher Weise zu dentalen Restaurationen verarbeitbar sein und die aus ihr hergestellten Restaurationen sollen neben einer sehr guten chemischen Beständigkeit auch über sehr gute mechanische und optische Eigenschaften verfügen.

Diese Aufgabe wird durch die Lithiumdisilikat-Apatit-Glaskeramik nach den Ansprüchen 1 bis 12 und 14 gelöst. Gegenstand der Erfindung sind ebenfalls die Lithiummetasilikat-Glaskeramik nach Anspruch 13 oder 14, das Verfahren nach Ansprüchen 15 und 16 sowie die Verwendung nach Ansprüchen 17 und 18.

Die erfindungsgemäße Lithiumdisilikat-Apatit-Glaskeramik zeichnet sich dadurch aus, dass sie Lithiumdisilikat als Hauptkristallphase und Apatit als weitere Kristallphase enthält und 3,0 bis 7,0, insbesondere 3,5 bis 6,0 Gew.-% K₂O enthält.

Mit dem Begriff "Hauptkristallphase" wird die Kristallphase bezeichnet, die gegenüber anderen Kristallphasen den höchsten Volumenanteil hat.

Überraschenderweise zeichnet sich die erfindungsgemäße Lithiumdisilikat-Apatit-Glaskeramik durch eine sehr hohe chemische Beständigkeit aus. Zur Bestimmung der chemischen Beständigkeit wurde die Glaskeramik gemäß ISO-Norm 6872 (2008) geprüft, indem der Masseverlust bei Lagerung in wäßriger Essigsäure festgestellt wurde. Die erfindungsgemäße Lithiumdisilikat-Apatit-Glaskeramik zeigte dabei insbesondere einen Masseverlust von weniger als 100 µg/cm², bevorzugt weniger als 90 und besonders bevorzugt weniger als 80 µg/cm² und ganz besonders bevorzugt weniger als 50 µg/cm².

Die Lithiumdisilikat-Apatit-Glaskeramik enthält vorzugsweise 60,0 bis 74,0, insbesondere 63,0 bis 71,0 Gew.-% SiO₂.

Auch ist es bevorzugt, dass die Lithiumdisilikat-Apatit-Glaskeramik 10,0 bis 20,0, insbesondere 11,0 bis 19,0 Gew.-% Li₂O enthält.

Das molare Verhältnis von SiO₂ zu Li₂O beträgt insbesondere 1.75 bis 3.0.

Weiter ist eine Lithiumdisilikat-Apatit-Glaskeramik bevorzugt, die 3,0 bis 7,0, insbesondere 4,0 bis 6,0 Gew.-% P₂O₅ enthält.

In einer weiteren bevorzugten Ausführungsform enthält die erfindungsgemäße Lithiumdisilikat-Apatit-Glaskeramik 0 bis 5,0, insbesondere 0 bis 4,5 Gew.-% CaO und 0 bis 4,0, insbesondere 0 bis 3,5 Gew.-% SrO enthält, wobei die kombinierte Menge an CaO und SrO 1,0 bis 6,0, insbesondere 1,5 bis 5,5 Gew.-% beträgt.

Auch ist eine Lithiumdisilikat-Apatit-Glaskeramik bevorzugt, die 0,1 bis 1,0, insbesondere 0,2 bis 0,5 Gew.-% F enthält.

Durch den Einsatz von Fluor ist die Bildung von Fluorapatit möglich. Es ist besonders bevorzugt, dass die erfindungsgemäße Glaskeramik Fluorapatit als Apatit enthält.

In einer bevorzugten Ausführungsform enthält die Lithiumdisilikat-Apatit-Glaskeramik auch 2,0 bis 7,0, insbesondere 3,0 bis 6,0 Gew.-% Oxid dreiwertiger Elemente und/oder weiteres Oxid vierwertiger Elemente.

Vorzugsweise ist das Oxid dreiwertiger Elemente ausgewählt aus der Gruppe von Al₂O₃, Y₂O₃, La₂O₃ und Mischungen davon. Besonders bevorzugt ist das Oxid dreiwertiger Elemente Al₂O₃. Ganz besonders bevorzugt enthält die erfindungsgemäße Lithiumsilikat-Apatit-Glaskeramik 3,0 bis 6,0 Gew.-% Al₂O₃.

Der Begriff "weiteres Oxid vierwertiger Elemente" bezeichnet Oxide vierwertiger Elemente mit Ausnahme von SiO₂. Beispiele für geeignete weitere Oxide vierwertiger Elemente sind ZrO₂, TiO₂, GeO₂ und Mischungen davon.

Weiter ist eine Lithiumdisilikat-Apatit-Glaskeramik bevorzugt, die mindestens eine und insbesondere alle folgenden Komponenten enthält:

| Komponente | Gew.-% |
|---|---|
| SiO₂ | 60,0 bis 74,0 |
| Li₂O | 10,0 bis 20,0 |
| P₂O₅ | 3,0 bis 7,0, |
| K₂O | 3,0 bis 7,0 |
| Ca0 | 0 bis 5,0 |
| SrO | 0 bis 4,0 |
| F | 0,1 bis 1,0 |
| Oxid dreiwertiger Elemente und/oder weiteres Oxid vierwertiger Elemente | 2,0 bis 7,0, |
| wobei | |
| CaO + SrO beträgt. | 1,0 bis 6,0 |

Die erfindungsgemäße Lithiumdisilikat-Apatit-Glaskeramik kann darüber hinaus noch Zusatzkomponenten enthalten, die insbesondere ausgewählt sind aus Färbemitteln und Fluoreszenzmitteln.

Beispiel für Färbemittel und Fluoreszenzmittel sind Oxide von d- und f-Elementen, wie z.B. die Oxide von Ti, V, Sc, Mn, Fe, Co, Ta, W, Ce, Pr, Nd, Tb, Er, Dy, Gd, Eu und Yb. Als Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden. Die Metallkolloide sind vorzugsweise in einer Menge von 0,005 bis 0,5 Gew.-% in der Glaskeramik enthalten.

In einer bevorzugten Ausführungsform enthält die erfindungsgemäße Lihtiumdisilikat-Apatit-Glaskeramik mehr als 20 Vol.-%, bevorzugt mehr als 25 Vol.-% und besonders bevorzugt mehr als 30 Vol.-% an Lithiumdisilikat-Kristallen, bezogen auf die gesamte Glaskeramik.

Die erfindungsgemäße Glaskeramik mit Lithiumdisilikat als Hauptkristallphase zeichnet sich durch besonders gute mechanische Eigenschaften aus und sie kann z.B. durch Wärmebehandlung eines entsprechenden Ausgangsglases oder eines entsprechenden Ausgangsglases mit Keimen oder einer entsprechenden Lithiummetasilikat-Glaskeramik gebildet werden.

Es hat sich überraschenderweise gezeigt, dass die erfindungsgemäße Lithiumdisilikat-Apatit-Glaskeramik über eine ausgezeichnet chemische Beständigkeit verfügt und zudem sehr gute mechanische und optische Eigenschaften aufweist. Sie ist damit den bekannten bioaktiven Lithiumsilikat-Apatit-Glaskeramiken überlegen. Die Kombination ihrer Eigenschaften erlaubt es sogar, sie als Dentalmaterial und insbesondere Material zur Herstellung von Dentalrestaurationen einzusetzen.

Die erfindungsgemäße Lithiumdisilikat-Apatit-Glaskeramik hat insbesondere eine Bruchzähigkeit, gemessen als K_{IC} Wert, von mindestens etwa 1,6 MPa•m^{0.5} und insbesondere mindestens etwa 1,8 MPa•m^{0.5}. Dieser Wert wurde mit dem Vicker's-Verfahren bestimmt und mittels Niihara-Gleichung berechnet. Weiter hat sie eine hohe biaxiale Bruchfestigkeit von bevorzugt 250 bis 550 MPa. Die biaxiale Bruchfestigkeit wurde gemäß ISO 6872 (2008) bestimmt.

Ebenfalls werden verschiedene Vorstufen mit entsprechender Zusammensetzung offenbart, aus denen die erfindungsgemäße Lithiumdisilikat-Apatit-Glaskeramik durch Wärmebehandlung hergestellt werden kann. Diese Vorstufen sind ein entsprechendes Ausgangsglas, ein entsprechendes Ausgangsglas mit Keimen und eine entsprechende Lithiummetasilikat-Glaskeramik.

Offenbart wird daher ebenfalls ein Ausgangsglas, das die Komponenten der erfindungsgemäßen Lithiumdisilikat-Apatit-Glaskeramik enthält.

Das Ausgangsglas enthält daher neben 3,0 bis 7,0 Gew.-% K₂O auch insbesondere geeignete Mengen an weiteren Komponenten, die zur Ausbildung der erfindungsgemäßen Glaskeramik mit Lithiumdisilikat als Hauptkristallphase und Apatit als weiterer Kristallphase erforderlich sind. Bevorzugt enthält es SiO₂ und Li₂O in Mengen, die die Ausbildung von Lithiumdisilikat ermöglichen. Weiter kann das Ausgangsglas auch noch andere Komponenten enthalten, wie sie oben für die erfindungsgemäße Lithiumdisilikat-Apatit-Glaskeramik angegeben sind. Es sind alle solche Ausführungsformen für die Komponenten des Ausgangsglases bevorzugt, die auch für die Komponenten der erfindungsgemäßen Lithiumdisilikat-Apatit-Glaskeramik als bevorzugt angegeben sind.

Ebenfalls offenbart wird ein solches Ausgangsglas, das Keime für die Kristallisation von Lithiummetasilikat, Lithiumdisilikat und/oder Apatit enthält.

Weiter betrifft die Erfindung eine Lithiummetasilikat-Glaskeramik, die die Komponenten der erfindungsgemäßen Lithiumdisilikat-Apatit-Glaskeramik enthält. Diese Lithiummetasilikat-Glaskeramik enthält daher neben 3,0 bis 7,0 Gew.-% K₂O auch insbesondere geeignete Mengen an weiteren Komponenten, die zur Ausbildung der erfindungsgemäßen Glaskeramik mit Lithiumdisilikat als Hauptkristallphase und Apatit als weiterer Kristallphase erforderlich sind. Weiter kann die Lithiummetasilikat-Glaskeramik auch noch andere Komponenten enthalten, wie sie oben für die erfindungsgemäße Lithiumdisilikat-Apatit-Glaskeramik angegeben sind. Es sind alle solche Ausführungsformen für die Komponenten der Lithiummetasilikat-Glaskeramik bevorzugt, die auch für die Komponenten der erfindungsgemäßen Lithiumdisilikat-Apatit-Glaskeramik als bevorzugt angegeben sind.

Durch Wärmebehandlung des Ausgangsglases können zunächst die weiteren Vorstufen Ausgangsglas mit Keimen und Lithiummetasilikat-Glaskeramik erzeugt werden. Durch Wärmebehandlung von einer dieser beiden weiteren Vorstufen kann dann die erfindungsgemäße Lithiumdisilikat-Apatit-Glaskeramik erzeugt werden. Es ist bevorzugt, die erfindungsgemäße Lithiumdisilikat-Apatit-Glaskeramik direkt durch Wärmebehandlung des Ausgangsglases mit Keimen zu bilden.

Es ist bevorzugt, das Ausgangsglas einer Wärmebehandlung bei einer Temperatur von 430 bis 750°C, insbesondere 430 bis 550°C, für eine Dauer von 5 bis 120 min, insbesondere 10 bis 60 min, zu unterwerfen, um das Ausgangsglas mit Keimen für die Kristallisation von Lithiummetasilikat, Lithiumdisilikat und/oder Apatit zu erzeugen.

Es ist weiter bevorzugt, das Ausgangsglas mit Keimen einer Wärmebehandlung bei einer Temperatur von mehr als 600°C für eine Dauer von 5 bis 120 min, insbesondere 10 bis 60 min, zu unterwerfen, um die Lithiummetasilikat-Glaskeramik oder die Lithiumdisilikat-Apatit-Glaskeramik herzustellen. Zur Herstellung der Lithiumdisilikat-Apatit-Glaskeramik erfolgt die Wärmebehandlung des Ausgangsglases mit Keimen besonders bevorzugt bei 700 bis 1000°C, insbesondere 750 bis 900°C, für eine Dauer von 5 bis 120 min, insbesondere 10 bis 60 min.

Die Erfindung betrifft ebenfalls ein Verfahren zur Herstellung der erfindungsgemäßen Lithiumdisilikat-Apatit-Glaskeramik, bei dem das Ausgangsglas, das Ausgangsglas mit Keimen oder die Lithiummetasilikat-Glaskeramik mindestens einer Wärmebehandlung im Bereich von 430° bis 1000°C unterzogen wird.

Die im erfindungsgemäßen Verfahren durchgeführte mindestens eine Wärmebehandlung kann auch im Rahmen eines Heißpressens oder Aufsinterns des Ausgangsglases, des Ausgangsglases mit Keimen oder der erfindungsgemäßen Lithiummetasilikat-Glaskeramik erfolgen.

In einer bevorzugten Ausführungsform umfasst das erfindungsgemäße Verfahren
(a) die Wärmebehandlung des Ausgangsglases bei einer Temperatur von 430 bis 550°C, um das Ausgangsglas mit Keimen zu bilden, und
(b) die Wärmebehandlung des Ausgangsglases mit Keimen bei einer Temperatur von 750 bis 950°C, um die Lithiumdisilikat-Apatit-Glaskeramik zu bilden.

Die Dauer der bei (a) und (b) durchgeführten Wärmebehandlungen beträgt insbesondere 5 bis 120 min und bevorzugt 10 bis 60 min.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Es ist ebenfalls möglich, die Schmelze erneut in Wasser zu geben, um ein Granulat herzustellen. Dieses Granulat kann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung unterzogen. Es ist bevorzugt, dass zunächst eine erste Wärmebehandlung durchgeführt wird, um ein Ausgangsglas mit Keimen herzustellen, welche zur Bildung von Lithiummetasilikat-, Lithiumdisilikat- und/oder Apatit-Kristallen geeignet sind. Das Glas mit Keimen wird dann üblicherweise mindestens einer weiteren Temperaturbehandlung bei einer höheren Temperatur unterworfen, um Kristallisation von Lithiummetasilikat, Lithiumdisilikat und/oder Apatit zu bewirken.

Zur Kristallisation von Lithiummetasilikat erfolgt die weitere Wärmebehandlung insbesondere bei einer Temperatur von mindestens 600°C. Zur Kristallisation von Lithiumdisilikat erfolgt die weitere Wärmebehandlung insbesondere bei einer Temperatur von mindestens 700°C. Zur Kristallisation von Apatit erfolgt die weitere Wärmebehandlung insbesondere bei einer Temperatur von mindestens 800°C.

Die erfindungsgemäßen Glaskeramiken und die oben beschriebenen Gläser liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Inlays, Onlays, Kronen, Veneers, Schalen oder Abutments, vorliegen.

Aus den erfindungsgemäßen Glaskeramiken und den oben beschriebenen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das oben beschriebene Ausgangsglas und insbesondere das oben beschriebene Ausgangsglas mit Keimen, die erfindungsgemäße Lihtiummetasilikat-Glaskeramik und die erfindungsgemäße Lithiumdisilikat-Apatit-Glaskeramik verwendet werden. Dabei können die Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das oben beschriebene Ausgangsglas, das oben beschriebene Ausgangsglas mit Keimen, die erfindungsgemäße Lithiummetasilikat-Glaskeramik und die erfindungsgemäße Lithiumdisilikat-Apatit-Glaskeramik verwendet werden. Dabei können die Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiummetasilikat-Glaskeramik oder die erfindungsgemäße Lithiumdisilikat-Apatit-Glaskeramik verwendet. Die Lithiumdisilikat-Apatit-Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich ist. Nach der maschinellen Bearbeitung eines solchen teilkristallisierten Materials wird dieses regelmäßig einer Wärmebehandlung bei höherer Temperatur und insbesondere 700 bis 1000°C und bevorzugt 750 bis 900°C unterzogen, um eine weitere Kristallisation von Lithiumdisilikat und Apatit hervorzurufen.

Allgemein kann nach der Herstellung der gewünscht geformten dentalen Restauration, z. B. durch Verpressen oder maschinelle Bearbeitung, diese insbesondere noch wärmebehandelt werden, um eingesetzte Vorläufer, wie Ausgangsglas, Ausgangsglas mit Keimen oder Lithiummetasilikat-Glaskeramik in Lithiumdisilikat-Apatit-Glaskeramik umzuwandeln oder die Kristallisation von Lithiumdisilikat und/oder Apatit zu steigern oder die Porosität, z.B. eines eingesetzten porösen Pulverpressling, zu vermindern.

Die erfindungsgemäßen Glaskeramiken und die oben beschriebenen Gläser eignen sich allerdings auch als Beschichtungsmaterial von z.B. Keramiken und Glaskeramiken. Die Erfindung ist daher ebenfalls auf die Verwendung der Gläser oder der erfindungsgemäßen Glaskeramiken zur Beschichtung von insbesondere Keramiken und Glaskeramiken gerichtet.

Die Erfindung betrifft auch ein Verfahren zur Beschichtung von Keramiken und Glaskeramiken, bei dem erfindungsgemäße Glaskeramiken oder Gläser auf die Keramik oder Glaskeramik aufgebracht und erhöhter Temperatur ausgesetzt wird.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird erfindungsgemäße Glaskeramik oder Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges die erfindungsgemäße Glaskeramik mit Lithiumdisilikat als Hauptkristallphase und Apatit als weiterer Kristallphase vorliegt, da eine solche Glaskeramik über besonders gute Eigenschaften verfügt.

Aufgrund der vorstehend geschilderten Eigenschaften der erfindungsgemäßen Glaskeramiken und der oben beschriebenen Gläser eignen sich diese insbesondere zum Einsatz in der Zahnheilkunde. Gegenstand der Erfindung ist daher auch die Verwendung der erfindungsgemäßen Glaskeramiken als Dentalmaterial und insbesondere zur Herstellung dentaler Restaurationen oder als Beschichtungsmaterial für dentale Restaurationen, wie Kronen, Brücken und Abutments.

Die Erfindung wird im Folgenden anhand von sie nichtbeschränkenden Beispielen näher erläutert.

### Beispiele

### Beispiele 1 bis 14 und Vergleich - Zusammensetzung und Kristallphasen

Es wurden insgesamt 14 Gläser und erfindungsgemäße Glaskeramiken mit der aus Tabelle I angegebenen Zusammensetzung über Erschmelzung entsprechender Ausgangsgläser und anschließende Wärmebehandlung zur gesteuerten Keimbildung und Kristallisation hergestellt.

Zum Vergleich wurde ebenfalls eine nicht erfindungsgemäße Glaskeramik erzeugt, die insbesondere keinerlei K₂O enthielt.

Die angewendeten Wärmebehandlungen zur gesteuerten Keimbildung und gesteuerten Kristallisation sind ebenfalls in Tabelle I angegeben. Dabei bedeuten
- T_{N} und t_{N}: Angewendete Temperatur und Zeit für Keimbildung
- Tₖ₁ und tₖ₁: Angewendete Temperatur und Zeit für Kristallisation von Lithiummetasilikat
- Tₖ₂ und tₖ₂: Angewendete Temperatur und Zeit für Kristallisation von Lithiumdisilikat und Apatit

Dazu wurden zunächst die Ausgangsgläser im 100 bis 200 g Massstab aus üblichen Rohstoffen bei 1400 bis 1500°C erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1450 bis 1550°C für 1 bis 3 h geschmolzen wurden.

Eine erste Wärmebehandlung der Ausgangsgläser bei einer Temperatur von 450 bis 470°C führte zur Bildung von Lithiumsilikat-Gläsern mit Keimen. Diese keimhaltigen Gläser kristallisierten durch eine weitere Wärmebehandlung bei 850°C zu Glaskeramiken mit Lithiumdisilikat als Hauptkristallphase und Apatit als weiterer Kristallphase, wie durch Röntgenbeugungsuntersuchungen festgestellt wurde. Es wurden daher Lithiumdisilikat-Apatit-Glaskeramiken erhalten.

Im Falle von Beispiel 4 führte die Wärmebehandlung des keimhaltigen Ausgangsglases bei einer Temperatur von lediglich 700°C zur Kristallisation von Lithiummetasilikat und damit Bildung einer Lithiummetasilikat-Glaskeramik. Diese Vorstufe wurde durch eine weitere Wärmebehandlung bei 850°C in die entsprechende Lithiumdisilikat-Apatit-Glaskeramik umgewandelt.

Die erzeugten erfindungsgemäßen Lithiumdisilikat-Apatit-Glaskeramiken zeigten eine ausgezeichnete chemische Beständigkeit gemäß ISO-Test 6872 (2008). Der Masseverlust bei Lagerung in wässriger Essigsäure betrug weniger als 100 µg/cm², insbesondere weniger als 50 µg/cm².

Demgegenüber zeigte die zum Vergleich hergestellte konventionelle Glaskeramik einen sehr hohen Masseverlust von 754 µg/cm² und damit eine sehr geringe chemische Beständigkeit. Sie ist zum Einsatz als restauratives Dentalmaterial nicht geeignet, welches in der Mundhöhle stetig mit Fluiden unterschiedlichster Zusammensetzung in Kontakt kommt.

Die erzeugten Lithiumdisilikat-Apatit-Glaskeramiken hatten auch hohe Bruchzähigkeiten, gemessen als kritischer Spannungsintensitätsfaktor K_{IC}, von mehr als 1,8 MPa•m^{0.5}.

Auch die Biaxialfestigkeit σ_{B} war mit mindestens 250 MPa hoch. Sie wurde gemäß Dentalnorm ISO 6872 (2008) an Prüfkörpern bestimmt, die durch maschinelle Bearbeitung der jeweiligen Lithiumdisilikat-Apatit-Glaskeramik hergestellt wurden. Zur Bearbeitung wurde eine CEREC®-InLab Maschine (Sirona, Bensheim) verwendet.

Die erzeugten Lithiumdisilikat-Apatit-Glaskeramiken und die als Vorstufe erzeugte Lithiummetasilikat-Glaskeramik konnten sehr gut maschinell in einem CAD/CAM-Verfahren oder durch Heißpressen in die Form verschiedener Dentalrestaurationen gebracht werden, die bei Bedarf noch mit einer Verblendung versehen wurden.

Ebenfalls konnten sie durch Heißpressen als Beschichtungen auf insbesondere Dentalrestaurationen aufgebracht werden, z.B. um diese in gewünschter Weise zu verblenden.

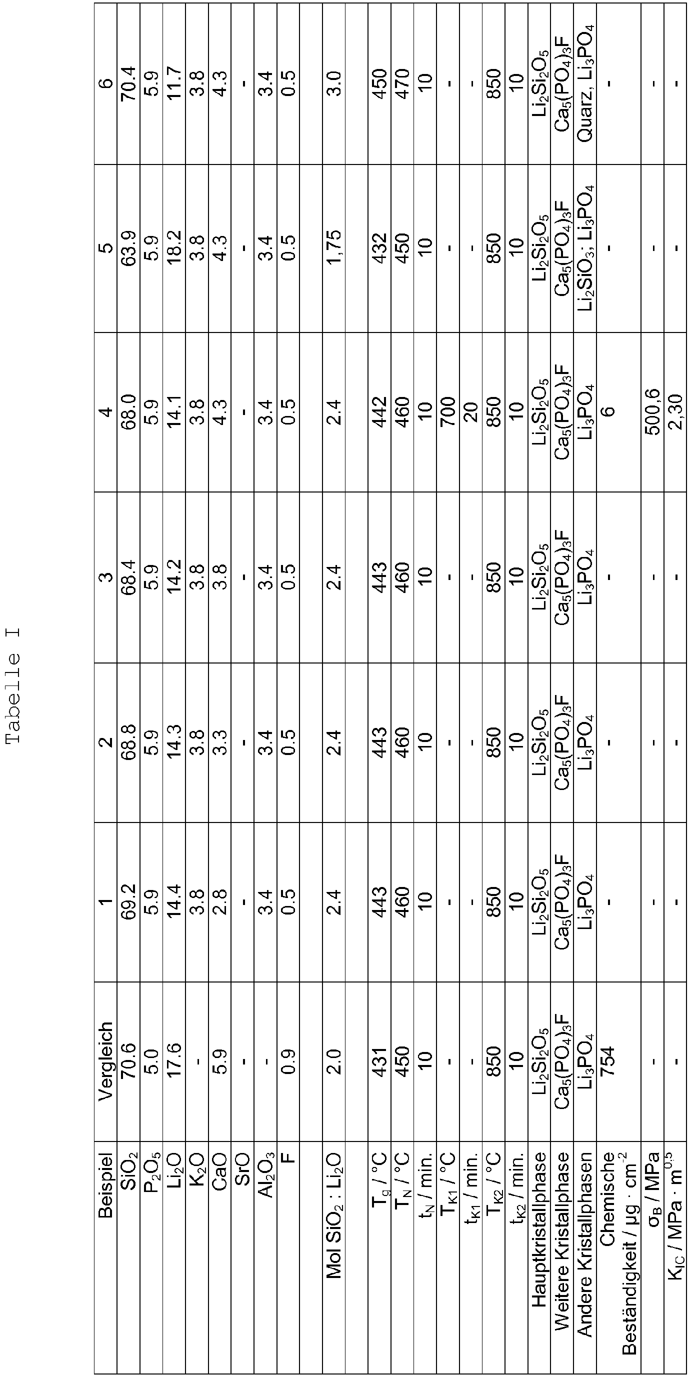

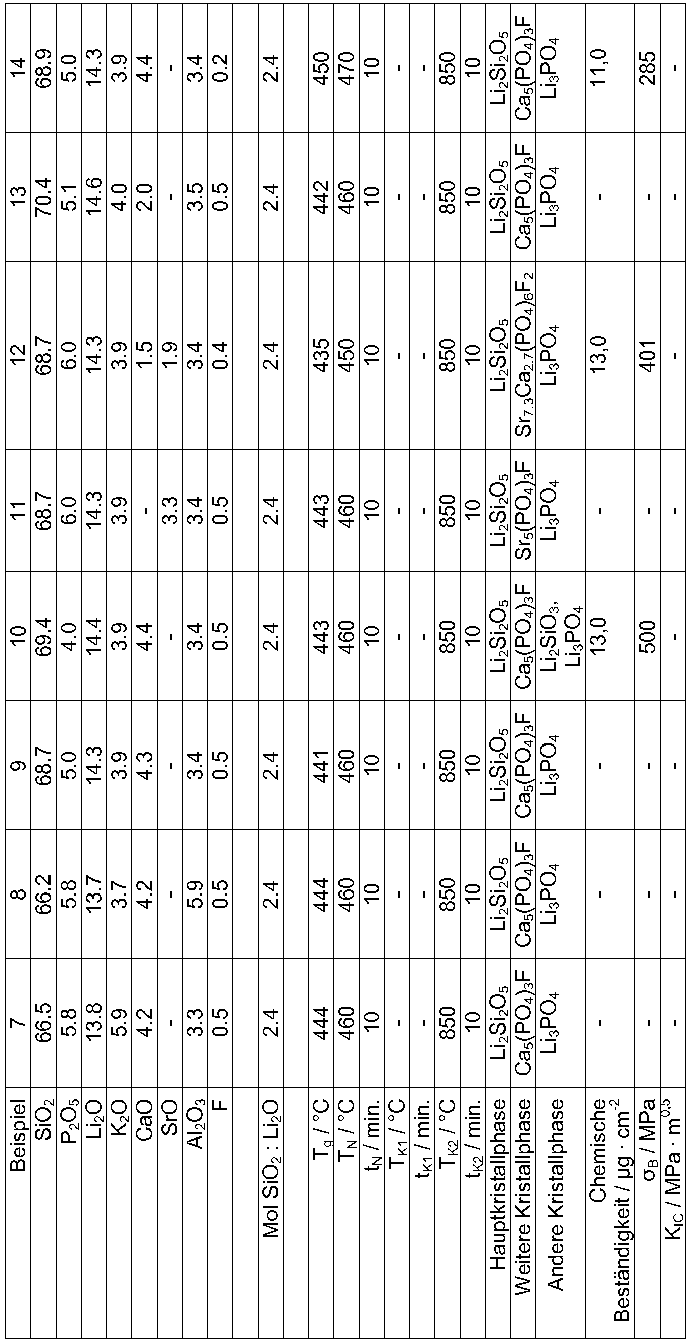

## Patentansprüche

1. Lithiumdisilikat-Apatit-Glaskeramik, die Lithiumdisilikat als Hauptkristallphase und Apatit als weitere Kristallphase enthält und die 3,0 bis 7,0, insbesondere 3,5 bis 6,0 Gew.% K₂O enthält.

2. Glaskeramik nach Anspruch 1, die 60,0 bis 74,0, insbesondere 63,0 bis 71,0 Gew.% SiO₂ enthält.

3. Glaskeramik nach Anspruch 1 oder 2, die 10,0 bis 20,0, insbesondere 11,0 bis 19,0 Gew.% Li₂O enthält.

4. Glaskeramik nach einem der Ansprüche 1 bis 3, die 3,0 bis 7,0, insbesondere 4,0 bis 6,0 Gew.-% P₂O₅ enthält.

5. Glaskeramik nach einem der Ansprüche 1 bis 4, die 0 bis 5,0, insbesondere 0 bis 4,5 Gew.% CaO und 0 bis 4,0, insbesondere 0 bis 3,5 Gew.-% SrO enthält, wobei die kombinierte Menge an CaO und SrO 1,0 bis 6,0, insbesondere 1,5 bis 5,5 Gew.-% beträgt.

6. Glaskeramik nach einem der Ansprüche 1 bis 5, die 0,1 bis 1,0, insbesondere 0,2 bis 0,5 Gew.-% F enthält.

7. Glaskeramik nach einem der Ansprüche 1 bis 6, die 2,0 bis 7,0, insbesondere 3,0 bis 6,0 Gew.-% Oxid dreiwertiger Elemente und/oder weiteres Oxid vierwertiger Elemente enthält.

8. Glaskeramik nach Anspruch 7, wobei das Oxid dreiwertiger Elemente ausgewählt ist aus der Gruppe von Al₂O₃, Y₂O₃, La₂O₃ und Mischungen davon und insbesondere Al₂O₃ ist.

9. Glaskeramik nach Anspruch 7 oder 8, wobei das weitere Oxid vierwertiger Elemente ausgewählt ist aus der Gruppe von ZrO₂, TiO₂, GeO₂ und Mischungen davon.

10. Glaskeramik nach einem der Ansprüche 1 bis 9, die mindestens eine und bevorzugt alle folgenden Komponenten enthält:
| Komponente | Gew.-% |
|---|---|
| SiO₂ | 60,0 bis 74,0 |
| Li₂O | 10,0 bis 20,0 |
| P₂O₅ | 3,0 bis 7,0, |
| K₂O | 3,0 bis 7,0 |
| CaO | 0 bis 5,0 |
| SrO | 0 bis 4,0 |
| F | 0,1 bis 1,0 |
| Oxid dreiwertiger Elemente und/oder weiteres Oxid vierwertiger Elemente | 2,0 bis 7,0, |
| wobei | |
| CaO + SrO beträgt. | 1,0 bis 6,0 |

11. Glaskeramik nach einem der Ansprüche 1 bis 10, die Fluorapatit als Apatit enthält.

12. Glaskeramik nach einem der Ansprüche 1 bis 11, die mehr als 20 Vol.-%, bevorzugt mehr als 25 Vol.-% und besonders bevorzugt mehr als 30 Vol.-% an Lithiumdisilikat-Kristallen aufweist.

13. Lithiummetasilikat-Glaskeramik, die die Komponenten der Glaskeramik nach einem der Ansprüche 1 bis 10 enthält.

14. Glaskeramik nach einem der Ansprüche 1 bis 12 oder Lithiummetasilikat-Glaskeramik nach Anspruch 13, wobei die Glaskeramik und die Lithiummetasilikat-Glaskeramik in Form von einem Pulver, einem Granulat, einem Rohling oder einer dentalen Restauration vorliegen.

15. Verfahren zur Herstellung der Glaskeramik gemäß einem der Ansprüche 1 bis 12, bei dem ein Ausgangsglas, das die Komponenten der Glaskeramik enthält, oder die Lithiummetasilikat-Glaskeramik gemäß Anspruch 13 mindestens einer Wärmebehandlung im Bereich von 430° bis 1000°C unterzogen wird

16. Verfahren nach Anspruch 15, bei dem
(a) das Ausgangsglas einer Wärmebehandlung bei einer Temperatur von 430 bis 550°C unterworfen wird, um Ausgangsglas mit Keimen zu bilden, und
(b) das Ausgangsglas mit Keimen einer Wärmebehandlung bei einer Temperatur von 750 bis 950°C unterworfen wird, um die Lithiumdisilikat-Apatit-Glaskeramik zu bilden.

17. Verwendung der Glaskeramik gemäß einem der Ansprüche 1 bis 12 oder 14 oder der Lithiummetasilikat-Glaskeramik gemäß Anspruch 13 oder 14 als Dentalmaterial, insbesondere zur Beschichtung dentaler Restaurationen und bevorzugt zur Herstellung dentaler Restaurationen.

18. Verwendung zur Herstellung dentaler Restaurationen nach Anspruch 17, wobei der Glaskeramik oder der Lithiummetasilikat-Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

## Claims

1. Lithium disilicate apatite glass-ceramic, which comprises lithium disilicate as main crystal phase and apatite as further crystal phase and which comprises 3.0 to 7.0, in particular 3.5 to 6.0 wt.-% K₂O.

2. Glass-ceramic according to claim 1, which comprises 60.0 to 74.0, in particular 63.0 to 71.0 wt.-% SiO₂.

3. Glass-ceramic according to claim 1 or 2, which comprises 10.0 to 20.0, in particular 11.0 to 19.0 wt.-% Li₂O.

4. Glass-ceramic according to any one of claims 1 to 3, which comprises 3.0 to 7.0, in particular 4.0 to 6.0 wt.-% P₂O₅.

5. Glass-ceramic according to any one of claims 1 to 4, which comprises 0 to 5.0, in particular 0 to 4.5 wt.-% CaO and 0 to 4.0, in particular 0 to 3.5 wt.-% SrO, wherein the combined amount of CaO and SrO is 1.0 to 6.0, in particular 1.5 to 5.5 wt.-%.

6. Glass-ceramic according to any one of claims 1 to 5, which comprises 0.1 to 1.0, in particular 0.2 to 0.5 wt.-% F.

7. Glass-ceramic according to any one of claims 1 to 6, which comprises 2.0 to 7.0, in particular 3.0 to 6.0 wt.-% oxide of trivalent elements and/or further oxide of tetravalent elements.

8. Glass-ceramic according to claim 7, wherein the oxide of trivalent elements is selected from the group of Al₂O₃, Y₂O₃, La₂O₃ and mixtures thereof and is in particular Al₂O₃.

9. Glass-ceramic according to claim 7 or 8, wherein the further oxide of tetravalent elements is selected from the group of ZrO₂, TiO₂, GeO₂ and mixtures thereof.

10. Glass-ceramic according to any one of claims 1 to 9, which comprises at least one and preferably all of the following components:
| Component | wt.-% |
|---|---|
| SiO₂ | 60.0 to 74.0 |
| Li₂O | 10.0 to 20.0 |
| P₂O₅ | 3.0 to 7.0 |
| K₂O | 3.0 to 7.0 |
| CaO | 0 to 5.0 |
| SrO | 0 to 4.0 |
| F | 0.1 to 1.0 |
| Oxide of trivalent elements and/or further oxide of tetravalent elements | 2.0 to 7.0 |
| | |
| wherein | |
| | |
| CaO + SrO is | 1.0 to 6.0. |

11. Glass-ceramic according to any one of claims 1 to 10, which comprises fluoroapatite as apatite.

12. Glass ceramic according to any one of claims 1 to 11, which comprises more than 20 vol.-%, preferably more than 25 vol.-% and particularly preferably more than 30 vol.-% lithium disilicate crystals.

13. Lithium metasilicate glass-ceramic, which comprises the components of the glass-ceramic according to any one of claims 1 to 10.

14. Glass-ceramic according to any one of claims 1 to 12 or lithium metasilicate glass-ceramic according to claim 13, wherein the glass-ceramic and the lithium metasilicate glass-ceramic are present in the form of a powder, a granulate, a blank or a dental restoration.

15. Process for the preparation of the glass-ceramic according to any one of claims 1 to 12, wherein a starting glass comprising the components of the glass-ceramic or the lithium metasilicate glass-ceramic according to claim 13 is subjected to at least one heat treatment in the range of from 430° to 1000°C.

16. Process according to claim 15, wherein
(a) the starting glass is subjected to a heat treatment at a temperature of from 430 to 550°C in order to form starting glass with nuclei, and
(b) the starting glass with nuclei is subjected to a heat treatment at a temperature of from 750 to 950°C in order to form the lithium disilicate apatite glass-ceramic.

17. Use of the glass-ceramic according to any one of claims 1 to 12 or 14 or the lithium metasilicate glass-ceramic according to claim 13 or 14 as dental material, in particular for coating dental restorations and preferably for the preparation of dental restorations.

18. Use for the preparation of dental restorations according to claim 17, wherein the glass-ceramic or the lithium metasilicate glass-ceramic is given, by pressing or machining, the shape of the desired dental restoration, in particular bridge, inlay, onlay, veneer, abutment, partial crown, crown or shell.

## Revendications

1. Vitrocéramique de disilicate de lithium et d'apatite, qui comporte du disilicate de lithium en tant que phase cristalline principale et de l'apatite en tant que phase cristalline supplémentaire, et qui contient de 3,0 à 7,0 % et en particulier de 3,5 à 6,0 % en poids de K₂O.

2. Vitrocéramique conforme à la revendication 1, qui comporte de 60,0 à 74,0 % et en particulier de 63,0 à 71,0 % en poids de SiO₂.

3. Vitrocéramique conforme à la revendication 1 ou 2, qui comporte de 10,0 à 20,0 % et en particulier de 11,0 à 19,0 % en poids de Li₂O.

4. Vitrocéramique conforme à l'une des revendications 1 à 3, qui comporte de 3,0 à 7,0 % et en particulier de 4,0 à 6,0 % en poids de P₂O₅.

5. Vitrocéramique conforme à l'une des revendications 1 à 4, qui comporte de 0 à 5,0 % et en particulier de 0 à 4,5 % en poids de CaO et de 0 à 4,0 % et en particulier de 0 à 3,5 % en poids de SrO, étant entendu que les quantités combinées de CaO et SrO en représentent de 1,0 à 6,0 % et en particulier de 1,5 à 5,5 % en poids.

6. Vitrocéramique conforme à l'une des revendications 1 à 5, qui comporte de 0,1 à 1,0 % et en particulier de 0,2 à 0,5 % en poids de fluor.

7. Vitrocéramique conforme à l'une des revendications 1 à 6, qui comporte de 2,0 à 7,0 % et en particulier de 3,0 à 6,0 % en poids d'un oxyde d'élément trivalent et/ou d'un autre oxyde d'élément tétravalent.

8. Vitrocéramique conforme à la revendication 7, dans laquelle l'oxyde d'élément trivalent est choisi dans l'ensemble formé par Al₂O₃, Y₂O₃, La₂O₃ et leurs mélanges, mais est en particulier Al₂O₃.

9. Vitrocéramique conforme à la revendication 7 ou 8, dans laquelle l'autre oxyde d'élément tétravalent est choisi dans l'ensemble formé par ZrO₂, TiO₂, GeO₂ et leurs mélanges.

10. Vitrocéramique conforme à l'une des revendications 1 à 9, qui comporte au moins l'un des composants suivants, mais de préférence les comporte tous :
| Composant | % en poids |
|---|---|
| SiO₂ | 60,0 à 74,0 |
| Li₂O | 10,0 à 20,0 |
| P₂O₅ | 3,0 à 7,0 |
| K₂O | 3,0 à 7,0 |
| CaO | 0 à 5,0 |
| SrO | 0 à 4,0 |
| F | 0,1 à 1,0 |
| Oxyde d'élément trivalent et/ou autre oxyde d'élément tétravalent | 2,0 à 7,0 |
| étant entendu que CaO + SrO | 1,0 à 6,0. |

11. Vitrocéramique conforme à l'une des revendications 1 à 10, qui comporte, en tant qu'apatite, de la fluoroapatite.

12. Vitrocéramique conforme à l'une des revendications 1 à 11, qui comporte plus de 20 % en volume, de préférence plus de 25 % en volume et mieux encore plus de 30 % en volume de cristaux de disilicate de lithium.

13. Vitrocéramique de métasilicate de lithium, qui comporte les composants de vitrocéramique conformes à l'une des revendications 1 à 10.

14. Vitrocéramique conforme à l'une des revendications 1 à 12, ou vitrocéramique de métasilicate de lithium conforme à la revendication 13, laquelle vitrocéramique, ou laquelle vitrocéramique de métasilicate de lithium, se présente sous la forme d'une poudre, d'un granulat, d'une ébauche ou d'une restauration dentaire.

15. Procédé de fabrication d'une vitrocéramique conforme à l'une des revendications 1 à 12, dans lequel on fait subir à un verre de départ qui comporte les composants de la vitrocéramique, ou à une vitrocéramique de métasilicate de lithium conforme à la revendication 13, au moins un traitement thermique à une température située dans l'intervalle allant de 430 à 1000 °C.

16. Procédé conforme à la revendication 15, dans lequel
a) on fait subir au verre de départ un traitement thermique à une température de 430 à 550 °C, pour qu'il se forme des germes au sein du verre de départ,
b) et on fait ensuite subir au verre de départ contenant ces germes un traitement thermique à une température de 750 à 950 °C, pour qu'il se forme une vitrocéramique de disilicate de lithium et d'apatite.

17. Utilisation d'une vitrocéramique conforme à l'une des revendications 1 à 12 ou d'une vitrocéramique de métasilicate de lithium conforme à la revendication 13 ou 14 en tant que matériau dentaire, en particulier pour le revêtement de restaurations dentaires et de préférence pour la fabrication de restaurations dentaires.

18. Utilisation pour la fabrication de restaurations dentaires, conforme à la revendication 17, dans laquelle on donne à la vitrocéramique, ou à la vitrocéramique de métasilicate de lithium, par compression ou par usinage à la machine, la forme de la restauration dentaire voulue, en particulier bridge, incrustation inlay ou onlay, facette fine (« veneer »), pilier, couronne partielle ou totale, ou facette écaille.
